# EUROPEAN PATENT APPLICATION

(11) **EP 4 112 635 A1**
(43) Date of publication of application: **04.01.2023**
(21) Application number: 20920921.2
(22) Date of filing: 20.11.2020
(51) Int. Cl.: C07K 14/165, C12N 15/50, G01N 33/68, G01N 33/569

(54) **SARS-COV-2 RECOMBINANT RBD PROTEIN, AND PREPARATION METHOD THEREFOR**

(30) Priority: 28.02.2020 CN 202010126715
(71) Applicant: Shenzhen Yhlo Biotech Co., Ltd., 518116 Shenzhen (CN)
(72) Inventor: LIN, Xiaotao, Shenzhen, Guangdong 518116 (CN); RUAN, Qiulian, Shenzhen, Guangdong 518116 (CN); XING, Zhihao, Shenzhen, Guangdong 518116 (CN); QIAN, Chungen, Shenzhen, Guangdong 518116 (CN); HU, Kunhui, Shenzhen, Guangdong 518116 (CN)
(74) Representative: Leonard, Thomas Charles
(86) International application number: PCT/CN2020/130468
(87) International publication number: WO 2021/169434

(57) **Abstract**

Disclosed are a recombinant RBD protein of SARS-CoV-2, and a method for preparing the same. The recombinant RBD protein has a signal polypeptide sequence, an RBD protein sequence, a transmembrane domain sequence and a tag sequence in order from N-terminus to C-terminus. The recombinant RBD protein may be expressed on cell membranes, which facilitates protein stability, enrichment and detection.

## Description

### TECHNICAL FIELD

The present disclosure relates to the technical field of protein expression, in particular to a recombinant RBD protein of SARS-CoV-2 and a method for preparing the same.

### BACKGROUND

Severe Acute Respiratory Syndrome Coronavirus 2 (SARS-CoV-2) is a subtype of Severe Acute Respiratory Syndrome-related coronavirus species belonging to genus *Betacoronavirus,* family *Coronaviridae.* SARS-CoV-2 is enveloped and contains positivesense single-stranded RNA. SARS-CoV-2 caused the coronavirus disease 2019 (COVID-19) outbreak in late 2019. Its gene sequence belongs to the same lineage as, but different clades form Severe Acute Respiratory Syndrome (SARS) virus and Middle East Respiratory Syndrome (MERS) virus. The National Health Commission of the People's Republic of China has designated the acute respiratory disease caused by infection by this virus as a Class B statutory infectious disease that is managed as Class A.

The spike protein is the most important surface membrane protein of the coronavirus and contains two subunits, S1 and S2. S1 mainly contains a receptor binding domain (RBD), which is responsible for recognizing cell receptors. The spike protein undertakes the function of the virus to bind membrane receptors of the host cells and to be fused with membrane of the host cells, thus is an important site in the host for antibody actions and a key target for vaccine design. The spike protein of SARS-CoV-2 interacts with human ACE2 to infect human respiratory epithelial cells.

In the prior art, the diagnosis of COVID-19 is mainly carried out using a nucleic acid detection kit. In the nucleic acid detection method, RNA is reverse-transcribed by a reverse transcriptase into DNA, which is then amplified through PCR under the action of DNA polymerase to enhance the signal, thereby showing the existence of viral nucleic acid. However, the novel coronavirus is an RNA virus in which RNA molecules are easily degraded during sample collection, storage and transportation, leading to false negative results in nucleic acid testing. The nucleic acid detection reaction takes about 2 hours for once and requires a fluorescence quantitative PCR instrument, which means that it takes a long time and has high requirement on equipment.

In the process of virus infection, a human body will be stressed to produce corresponding antibodies to resist the infection. Therefore, an immunodiagnostic method to determine whether or not one is infected with the virus by detecting the presence of antibodies may be used as a good supplement to the nucleic acid detection method. Antibodies produced by the human body can exist stably in the blood, has high specificity and can be fast detected. RBD protein as SARS-CoV-2 receptor binding domain protein can stimulate the body to produce a large number of specific antibodies, but there is no purification method with specificity and high-yield for the RBD protein of SARS-CoV-2 in the prior art. Therefore, the detection of COVID-19-related antibodies using the recombinant RBD protein prepared in the prior art tends to be inaccurate, which will interfere with the detection results.

In view of this, the present disclosure is proposed.

### SUMMARY

A The present disclosure relates to a recombinant RBD protein of SARS-CoV-2 comprising a signal peptide sequence, an RBD protein sequence as set forth in SEQ ID NO: 1, a transmembrane domain sequence as set forth in SEQ ID NO: 2 and a tag sequence as set forth in SEQ ID NO:3 in order from N-terminus to C-terminus.

According to another aspect of the present disclosure, the present disclosure also relates to a method for preparing the RBD protein of SARS-CoV-2, including:
a) culturing the host cell as described above under a suitable condition, and collecting a lysate of the host cell and/or culture solution;
b) isolating the RBD protein from the fraction obtained in a) using a ligand specific for the tag sequence.

In other embodiments, the method for preparing the RBD protein of SARS-CoV-2 includes:
a) culturing the host cell as described above under a suitable condition, and collecting the host cell;
b) co-incubating the host cell with a membrane protein dissociation reagent; and
c) separating the incubated liquid into solid and liquid fractions, and collecting the RBD protein in the supernatant.

The RBD protein sequence as set forth in SEQ ID NO: 1 is a conserved sequence of SARS-CoV-2, which is beneficial to improve the positive rate as well as the sensitivity and specificity for detection. The transmembrane domain and the tag peptide are linked to the C-terminus of the RBD protein. On the one hand, the transmembrane domain keeps the recombinant protein on the cell membrane, which is conducive to the stability of the protein and facilitates the collection of cells by centrifugation and the enrichment of proteins; on the other hand, the intracellular tag peptide of at the C-terminus of RBD protein may be captured by corresponding tag antibody, eliminating the step for protein purification. The signal peptide guides RBD protein to be extracellularly secreted, resulting in post-translational modification, which is conducive to the formation of a correct spatial structure. Therefore, the fusion with the transmembrane domain and the C-terminal tag peptide fragment at the C-terminus of the RBD protein is beneficial to the stability of the recombinant protein, and can further improve the sensitivity and specificity for detection in combination with the use of a ligand specific for the tag sequence.

The present disclosure further provides a nucleic acid, a vector and a host cell related to the recombinant RBD protein.

The present disclosure further provides a composition obtained by purification by the method as described above.

The present disclosure also relates to a reagent or kit for detecting an antibody against RBD protein of SARS-CoV-2, comprising the recombinant RBD protein as described above or the composition as described above.

### BRIEF DESCRIPTION OF THE DRAWINGS

To describe the technical solutions of the embodiments of the present disclosure or the prior art more clearly, the following briefly introduces the accompanying drawings required for describing the embodiments or the prior art. Apparently, the accompanying drawings in the following description show some embodiments of the present disclosure, and persons of ordinary skill in the art may also derive other drawings from these accompanying drawings without creative efforts.

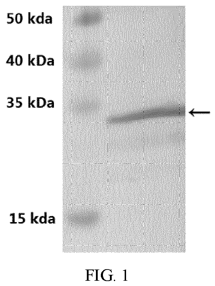

FIG. 1 is a Western blotting (WB) image of a transfected cell sample in one embodiment of the present disclosure.

### DETAILED DESCRIPTION OF THE EMBODIMENTS

Reference will now be provided in detail to the embodiments of the present disclosure, one or more examples of which are described below. Each example is provided by way of illustration rather than limitation to the present disclosure. In fact, it will be apparent to those skilled in the art that various modifications and variations can be made to the present disclosure without departing from the scope or spirit of the present disclosure. For example, features illustrated or described as part of one embodiment can be used in another embodiment to produce a further embodiment.

Such modifications and changes falling within the scope of the appended claims and the scope of equivalency of the claims are therefore intended to be embraced in the present disclosure. Other objects, features and aspects of the present disclosure are disclosed in or are apparent from the following detailed description. It should be understood by those of ordinary skill in the art that the present discussion is a description of exemplary embodiments only, and is not intended to limit the broader aspects of the present disclosure.

The present disclosure relates to a recombinant RBD protein of SARS-CoV-2 comprising a signal peptide sequence, an RBD protein sequence as set forth in SEQ ID NO: 1, a transmembrane domain sequence as set forth in SEQ ID NO: 2 and a tag sequence as set forth in SEQ ID NO:3 in order from N-terminus to C-terminus.

In some embodiments, the signal peptide sequence has a sequence as set forth in SEQ ID NO:4.

The present disclosure also provides a nucleic acid capable of expressing the recombinant RBD protein as described above.

In some embodiments, the nucleic acid has a sequence as set forth in SEQ ID NO:5.

The present disclosure also provides a vector comprising the nucleic acid as described above.

The term "vector" refers to a nucleic acid carrier into which polynucleotides may be inserted. When a vector enables the expression of a protein encoded by a polynucleotide inserted therein, the vector is called an expression vector. The vector may be introduced into a host cell through transformation, transduction or transfection, so that the genetic material element it carries is expressed in the host cell. Vectors are well known to those skilled in the art, including, but not limited to, plasmids; phagemids; cosmids; artificial chromosomes, such as yeast artificial chromosomes (YACs), bacterial artificial chromosomes (BACs) or PI-derived artificial chromosomes (PACs); bacteriophages, such as λ bacteriophage or M13 bacteriophage and animal viruses, etc. The animal viruses that may be used as vectors include, but are not limited to: retroviruses (including lentiviruses), adenoviruses, adeno-associated viruses, herpes viruses (such as herpes simplex virus), poxviruses, baculoviruses, papillomaviruses, and papovaviruses (such as SV40). In some embodiments, the vector described in the present disclosure comprises regulatory elements, such as enhancers, promoters, internal ribosome entry sites (IRES), and other expression control elements (such as transcription termination signals, or polyadenylation signal and poly (U) sequence, etc.) commonly used in genetic engineering. In a specific embodiment, the vector is pcDNA3.1(+).

The present disclosure also provides a host cell incorporating the nucleic acid as described above in genome thereof.

The term "host cell" refers to cells into which a vector can be introduced, including, but not limited to, prokaryotic cells, such as *Escherichia coli* (*E.coli*) or *Bacterium subtilis,* etc.; fungal cells, such as yeast cells or *Aspergillus,* etc.; insect cells, such as S2 drosophila cells or Sf9, etc.; or animal cells, such as fibroblasts, CHO cells, COS cells, NSO cells, HeLa cells, BHK cells, HEK 293 cells or human cells, etc.

The host cell is preferably a prokaryotic cell, preferably *E. coli,* more preferably *E. coli* TOP10 strain.

In some embodiments, the present disclosure also relates to a method for preparing the RBD protein of SARS-CoV-2 including:
a) culturing the host cell as described above under a suitable condition, and collecting a lysate of the host cell and/or culture solution;
b) isolating the RBD protein from the fraction obtained in a) using a ligand specific for the tag sequence.

In the present disclosure, the ligand specific for the tag sequence is a substance that is capable of specifically binding to the tag sequence, for example, an agglutin of the tag sequence, a nucleic acid aptamer that binds to the tag sequence, or an antibody and antibody fragment that bind to the tag sequence. Those skilled in the art will understand that the use of the term "specificity" means that other biomolecules present in the sample do not bind significantly to the tag sequence. The specific ligand has, for example, an affinity of at least 10⁶ l/mol for the tag sequence, preferably 10⁷ l/mol, 10⁸ l/mol, or more preferably 10⁹ l/mol for target molecule.

The ligand specific for the tag sequence may be bound to a solid phase and thus can be separated from the fraction obtained in a), as is familiar to those skilled in the art. The solid phase may be approximately spherical particulate matter.

In some embodiments, the solid phase is magnetic microspheres.

A particle size of the magnetic microspheres may be 0.2 µm to 5 µm.

The particle size of the magnetic microspheres may also be selected from 0.5 µm, 1 µm, 2 µm, 3 µm, or 4 µm.

Specifically, the magnetic microspheres may be Fe₃O₄ microspheres.

The method may further include a step of separating the ligand specific for the tag sequence from the tag sequence. The method of separation may be selected according to the nature of the ligand, which is easy for those skilled in the art. For example, the method of separation may be an acid pickling.

In other embodiments, the method for preparing the RBD protein of SARS-CoV-2 includes:
a) culturing the host cell as described above under a suitable condition, and collecting the host cell;
b) co-incubating the host cell with a membrane protein dissociation reagent; and
c) separating the incubated liquid into solid and liquid fractions, and collecting the RBD protein in the supernatant.

In some embodiments, main active ingredients of the membrane protein dissociation reagent includes 0.7%~1.3% of Triton X-100.

In some embodiments, the membrane protein dissociation reagent contains 7 mM~13 mM Na₂HPO₄ and 40 mM~50 mM NaCl.

In some embodiments, the membrane protein dissociation reagent contains 9 mM~11 mM Na₂HPO₄ and 43 mM~47 mM NaCl.

In some embodiments, the membrane protein dissociation reagent has a pH of 7.2 ~7.6, and a pH of 7.3, 7.4, 7.5 may also be selected.

In some embodiments, a method for separating the incubated liquid into solid and liquid fractions is centrifugation.

In some embodiments, the centrifugation is performed at 14000 g∼18000 g for 12 min~18 min.

In some embodiments, the centrifugation is performed at 15000 g~17000 g for 14min~16min.

The present disclosure further provides a composition obtained by purification by the method as described above.

According to yet another aspect of the present disclosure, the present disclosure also relates to a reagent or kit for detecting an antibody against RBD protein of SARS-CoV-2, comprising the recombinant RBD protein as described above or the composition as described above.

The antibody against RBD protein of SARS-CoV-2 is preferably derived from humans, and may also be derived from other potential hosts containing SARS-CoV-2, such as bats, pangolins, paguma larvatas, or other mammals.

The type of the antibody may be selected from IgM, IgE, IgA, IgD, or IgG.

The embodiments of the present disclosure will be described in detail below in combination with examples.

### Example

The following substances were selected: an RBD protein of the novel coronavirus spike protein having the following amino acid sequence: 
NITNLCPFGEVFNATRFASVYAWNRKRISNCVADYSVLYNSASFSTFKCY
GVSPTKLNDLCFTNVYADSFVIRGDEVRQIAPGQTGKIADYNYKLPDDFTGCVIA
WNSNNLDSKVGGNYNYLYRLFRKSNLKPFERDISTEIYQAGSTPCNGVEGFNCY
FPLQSYGFQPTNGVGYQPYRVVVLSFELLHAPATVCGPKKST (SEQ ID NO：1);
a signal peptide having the following amino acid sequence:
   MFVFLVLLPLVSSQCV (SEQ ID NO: 4);
a transmembrane domain having the following amino acid sequence:
   ELWLVLVAVGAGLLLLGLIILLL (SEQ ID NO: 2);
a tag peptide having the following amino acid sequence:
   WGQGGTHGQWNKPSKP (SEQ ID NO: 3).

The signal peptide, the RBD protein, the transmembrane domain and the tag peptide were connected in order and fused to form an RBD recombinant antigen. The DNA sequence encoding the complete fusion protein was:

ATGTTTGTTTTTCTTGTTTTATTGCCACTAGTCTCTAGTCAGTGTGTTAA
TATTACAAACTTGTGCCCTTTTGGTGAAGTTTTTAACGCCACCAGATTTGCATC
TGTTTATGCTTGGAACAGGAAGAGAATCAGCAACTGTGTTGCTGATTATTCTGT
CCTATATAATTCCGCATCATTTTCCACTTTTAAGTGTTATGGAGTGTCTCCTACT
AAATTAAATGATCTCTGCTTTACTAATGTCTATGCAGATTCATTTGTAATTAGAG
GTGATGAAGTCAGACAAATCGCTCCAGGGCAAACTGGAAAGATTGCTGATTAT
AATTATAAATTACCAGATGATTTTACAGGCTGCGTTATAGCTTGGAATTCTAACA
ATCTTGATTCTAAGGTTGGTGGTAATTATAATTACCTGTATAGATTGTTTAGGAA
GTCTAATCTCAAACCTTTTGAGAGAGATATTTCAACTGAAATCTATCAGGCCGG
TAGCACACCTTGTAATGGTGTTGAAGGTTTTAATTGTTACTTTCCTTTACAATC
ATATGGTTTCCAACCCACTAATGGTGTTGGTTACCAACCATACAGAGTAGTAGT
ACTTTCTTTTGAACTTCTACATGCACCAGCAACTGTTTGTGGACCTAAAAAGT
CTACTGAGCTGTGGCTGGTGCTGGTGGCCGTGGGCGCTGGACTGCTGCTGCT
GGGACTGATCATCCTGCTGCTGTGGGGTCAAGGCGGTACACATGGTCAATGGA
ACAAGCCTTCGAAGCCGTGA (SEQ ID NO：5).

The DNA encoding the recombinant RBD protein was obtained by gene synthesis (General Biosystems (Anhui) Co., Ltd.). The DNA encoding the RBD recombinant antigen had a HindIII site and a Kozak sequence upstream and an EcoRI site downstream. The DNA was digested with the corresponding restriction endonucleases and ligated into a Shuttle expression vector pcDNA3.1(+) digested with HindIII and EcoRI enzymes to obtain a recombinant plasmid pcDNA3.1(+)-RBD.

The recombinant plasmid was transformed into *E. coli* strain Top 10, and a single clone was picked, inoculated into 100 mLof LB medium containing 100 µg/mL ampicillin, and cultured overnight at 200 rpm at 37°C for extraction of the recombinant plasmid. The recombinant plasmid was extracted using an endotoxin-free plasmid extraction kit (Tiangen Biotech (Beijing) Co., Ltd., DP120). 293T cells were plated on a dish and cultured to about 85% confluence for transfection with the recombinant plasmid pcDNA3.1(+)-RBD.

A dish of cells was transfected with 10 µg of plasmid DNA in a mass ratio of the plasmid DNA to PEI of 1:5. After the plasmid DNA and PEI were each mixed well with 250 µL of PBS, the two mixtures were vortexed and mixed for 1 min. After standing at room temperature for 15 min, the two mixtures were evenly added dropwise to a cell culture dish. After well mixing, the cell culture dish was then placed in an incubator for 48 h. The transfected cells were scraped off, resuspended in PBS and washed twice, and a dish of cells was resuspended with 1 mL of buffer (Buffer A) of 10 mM Na₂HPO₄/50 mM NaCl/1% Triton X-100, pH 7.4, and then placed at 4°C for extraction for 30 min. The extract was centrifuged at 16,000 g for 15 min. The supernatant was the extracted recombinant RBD antigen, and stored at -20°C for later use.

It was confirmed from by western blotting assay (WB) that the target protein obtained through the above extraction steps was expressed, and the target protein was detected to be expressed. Because the RBD protein has glycosylation sites, the image from western blotting showed a larger relative molecular weight than the theoretical relative molecular weight. The electrophoretic results are shown in FIG. 1.

The respective technical features of the above-mentioned embodiments can be combined arbitrarily. For the sake of brevity, not all the possible combinations of each of the technical features in the above embodiments are described. However, all of the combinations of these technical features should be considered as within the scope of this disclosure, as long as such combinations do not contradict with each other.

The above-mentioned embodiments are merely illustrative of several embodiments of the present disclosure, which are described specifically and in detail, but should not to be construed as limiting the scope of the present disclosure. It should be noted that, for those ordinary skilled in the art, several variations and improvements may be made without departing from the concept of the present disclosure, and all of which are within the protection scope of the present disclosure. Therefore, the protection scope of the present disclosure shall be defined by the appended claims.

## Claims

1. A recombinant RBD protein of SARS-CoV-2, comprising a signal peptide sequence, an RBD protein sequence as set forth in SEQ ID NO: 1, a transmembrane domain sequence as set forth in SEQ ID NO: 2 and a tag sequence as set forth in SEQ ID NO:3 in order from N-terminus to C-terminus,
wherein, optionally, the signal peptide sequence has a sequence as set forth in SEQ ID NO:4.

2. A nucleic acid capable of expressing the recombinant RBD protein of claim 1, wherein, optionally, the nucleic acid has a sequence as set forth in SEQ ID NO:5.

3. A vector comprising the nucleic acid of claim 2.

4. A host cell incorporating the nucleic acid of claim 2 in genome thereof.

5. A method for preparing an RBD protein of SARS-CoV-2 comprising:
a) culturing the host cell of claim 4 under a suitable condition, and collecting a lysate of the host cell and/or culture solution;
b) isolating the RBD protein from the fraction obtained in a) using a ligand specific for the tag sequence;
wherein, optionally, the ligand specific for the tag sequence is an antibody.

6. A method for preparing an RBD protein of SARS-CoV-2 comprising:
a) culturing the host cell of claim 4 under a suitable condition, and collecting the host cell;
b) co-incubating the host cell with a membrane protein dissociation reagent; and
c) separating the incubated liquid into solid and liquid fractions, and collecting the RBD protein in the supernatant.

7. The method according to claim 6, wherein main active ingredients of the membrane protein dissociation reagent comprises 0.7%~1.3% of Triton X-100;
optionally, the membrane protein dissociation reagent contains 7 mM~13 mM Na₂HPO₄ and 40 mM~50 mM NaCl, pH=7.2~7.6.

8. The method according to claim 6, wherein a method for separating the incubated liquid into solid and liquid fractions is centrifugation.

9. A composition prepared by purification by the method of any one of claims 5 to 8.

10. A reagent or kit for detecting an antibody against RBD protein of SARS-CoV-2, comprising the recombinant RBD protein of claim 1, or the composition of claim 9.
